# EUROPEAN PATENT SPECIFICATION

(11) **EP 2 723 283 B1**
(45) Date of publication and mention of the grant of the patent: **01.08.2018**
(21) Application number: 11730905.4
(22) Date of filing: 27.06.2011
(51) Int. Cl.: A61F 9/008

(54) **APPARATUS FOR EYE SURGERY**
VORRICHTUNG FÜR DIE AUGENCHIRURGIE
APPAREIL POUR UNE CHIRURGIE DE L'OEIL

(43) Date of publication of application: 30.04.2014
(73) Proprietor: WaveLight GmbH, 91058 Erlangen (DE)
(72) Inventor: VOGLER, Klaus, 99444 Blankenhain (DE); DONITZKY, Christof, 90542 Eckental/Eschenau (DE)
(74) Representative: Katérle, Axel
(86) International application number: PCT/EP2011/003154
(87) International publication number: WO 2013/000487

(56) References cited:
- EP-B1- 1 731 120
- WO-A1-2008/098388
- DE-A1-102005 001 249
- US-A1- 2010 324 542

## Description

The present invention relates to laser-assisted operations on the human eye that include treatment of the eye by laser and further operation tasks to be performed in a sterile environment.

A laser-assisted cataract operation (i.e. the treatment of a cataract through implanting of an artificial lens in the eye) is an example of a form of operation in which, after the use of a laser, other operation devices are additionally used in order to complete the operation. The laser can be used to produce incisions, for example in order to open the anterior capsule region of the human eye (capsulorhexis) and to produce lateral incisions at the limbus edge so as to enable the human lens to be removed, an artificial lens to be inserted and the instruments required in this case to be introduced. The laser can also be used to prefragment the crystalline human lens, i.e. to divide it into segments, which can then be more easily liquified by phacoemulsification and aspirated. For this purpose, the laser can use ultrashort-pulse, focussed laser radiation, the laser pulses giving rise to photodisruptions in the irradiated tissue as a result of a laser-induced dielectric breakdown. Concatenation of such photodisruptions enables a multiplicity of incision shapes to be produced intraocularly. The pulse durations of the laser pulses can be, for example, in the picosecond, femtosecond or attosecond range, but shorter or longer pulse durations are also conceivable within the scope of the invention, provided that they can ensure the desired photodisruptive effect.

Clearly, laser-assisted cataract operations are only one possible form of operation within the scope of the invention. In general, the invention is suitable for any intraocular operations with laser assistance wherein, after the laser treatment, there are additionally required further operation tasks that absolutely must be performed under sterile-room conditions in order to prevent germs from entering open wounds of the eye.

In the case of a typical laser-assisted cataract operation, the laser treatment of the eye is performed first, in a first operation room, which is usually non-sterile. The patient lies on a treatment couch, the eye to be treated being immovably coupled, in a manner known per se, by means of an adapter (patient interface), to a focussing objective lens of the laser system used.

After completion of laser treatment, the patient is transferred by the medical personnel to another bed and brought into a second, separate operation room, in which conditions are sterile and in which the appliances and instruments necessary for the extraction of the crystalline lens and for the implantation of the artificial lens are available. It is necessary in this case to transfer the patient to another bed, i.e. the patient must get up from the couch in the first operation room, walk into the second operation room and lie on another couch there. Similarly, it is also necessary for the physician performing the treatment to go into the second operation room. Because conditions there are sterile, it is necessary for the physician to undergo usual disinfection measures and change his gloves and, if appropriate, also his clothes, before he may enter the second operation room.

This procedure (room change, transfer of patient to another bed, changing of clothes and disinfection by medical personnel) is cumbersome and time-consuming, and is also stressful for the patient, owing to the intermediate interruption in the course of the operation. These disadvantages impede the increasing advance of laser in cataract operations, although the advantages of the use of a laser in such an operation are significant in comparison with performance of an operation without laser.

Patent application DE 10 2005 001 249 A1 discloses a laser treatment unit for performing eye surgery comprising a contact glass which can be placed onto the eye and via which a treatment laser beam passes. A safety mechanism is provided that displaceably holds the contact glass in such a manner that the contact glass retreats when the contact glass is subjected to the action of a force contrary to the direction of incidence of the laser beam. The safety mechanism enables this retreating when a force is greater than a force limit value and holds the contact glass in a fixed manner when the force is less than the force limit value.

Patent application WO 2008/098388 A1 discloses an ophthalmological apparatus for breakdown of eye tissue comprising a base station with a light source for generating light pulses. A support arm, with an application head that can be placed onto an eye, is mounted on the base station. The light pulses are transmitted from the base station to the application head through an optical transmission system. The application head has a light projector for focused projection of the light pulses for punctiform breakdown of eye tissue. The support arm is of rigid design with horizontal orientation and has a hinge with a horizontally oriented rotation axis, the hinge being mounted in such a way that the application head can be placed onto the eye with a rotation extending about the rotation axis. The hinge permits controlled manual docking of the application head and light projector onto the eye in a vertical direction, via a rotation movement that is easy to perform and has minimal mechanical friction.

It is an object of the invention to indicate a way in which, in the case of laser-assisted intraocular eye operations that are to be performed, at least partially, in a sterile environment, the course of the treatment can be shortened and inconvenience to the patient can be reduced.

To achieve this object, the invention proposes an apparatus for eye surgery according to claim 1. Preferred embodiments of the apparatus are recited in the dependent claims.

In an example, there is provided an apparatus for eye surgery comprising a stand having a stand base that is movable or realized for mounting on a wall or ceiling, and having a stand arm arrangement that is manually adjustable, at least partially, relative to the stand base; an operation microscope attached to the stand arm arrangement; and a laser appliance, which provides pulsed, focussed laser radiation having radiation properties suited to the application of incisions in the human eye, the laser appliance having a laser source and a laser treatment head that is attached to the stand arm arrangement and emits the laser radiation, a flexible transmission fibre or a jointed beam transport arm being provided for the purpose of transporting the laser radiation to the laser treatment head, the laser treatment head being positioned or positionable in an observation beam path of the operation microscope and providing a passage for an observation beam going along the observation beam path.

An eye-surgery apparatus designed in such a manner enables a laser-assisted intraocular eye operation, for instance a cataract operation, to be performed at one operation location without a change of room. This avoids transfer of the patient to another bed, shortens the duration of the operation and, since the course of the operation is more convenient, because it is not interrupted, allows the expectation of better treatment results. The entire operation, including the laser treatment, can be performed in a sterile region of a single operation room, the operation room being easily cleaned and re-sterilized after completion of the
operation, owing to the stand being movable or mounted on a wall or ceiling. If required, sterile covers (e.g. cover films) can be provided, for example in order to cover particular parts (modules) of the eye-surgery apparatus, for instance the microscope, the laser treatment head and/or a swivel arm of the stand. Particularly in the case of use of such covers, the stand, with the components fastened thereto, can remain in the operation room.

The invention allows a laser scalpel to be integrated into the usual device system for a cataract operation or other intraocular eye operation. The connection of the laser source generating the radiation to the laser treatment head via a flexible transmission fibre or a jointed beam transport arm (mirror jointed arm) enables the laser treatment head, with a patient adapter attached thereto, to be moved, if required, into the conventional manual operation space of the physician. Through an observation passage in the laser treatment head, the physician performing the treatment, or an assistant, can observe the eye through the operation microscope for the purpose of performing the laser treatment. The laser source itself, which comprises, for example, a fibre laser or other solid-state laser, can be disposed at a certain distance from the sterile working region, for instance in a semi-sterile region of the operation room, but, alternatively, it can also be disposed in the sterile region. Expediently, the laser treatment head remains coupled to the stand, only a patient adapter, which is attached to the laser treatment head and via which the eye of the patient can be coupled to the laser treatment head, being detachable, such that it can be exchanged between successive operations and replaced by a new, sterile patient adapter.

In one design, it is conceivable for the laser treatment head to remain in the observation beam path of the operation microscope, not only for the laser treatment, but also in the case of subsequent operation tasks (e.g. extraction of the human lens, implantation of an artificial lens), the observation passage in the laser treatment head affording the physician the necessary view of the eye also in these subsequent operations tasks. For such a design, the operation microscope and the laser treatment head are coupled or can be coupled to one another relative to the stand base for the purpose of common positional adjustment. After the laser treatment, the physician must then be able to raise the laser treatment head from the eye, to enable the patient adapter to be removed. However, the laser treatment head can remain between the operation microscope and the eye, it being possible to ensure, through appropriate setting of the stand, that there is sufficient space between the eye and the treatment head, in order that the physician can perform the remaining tasks - observed through the operation microscope - in an unimpeded manner.

In another design, it is conceivable for the laser treatment head to be moved away out of the observation beam path of the operation microscope after the laser treatment, i.e. not to remain between the operation microscope and the eye during the subsequent operation tasks. For this purpose, the operation microscope and the laser treatment head are positionally adjustable relative to one another, in such a way that the laser treatment head can be moved into and out of the observation beam path of the operation microscope. The stand arm arrangement in this case can have a first arm unit, to which the operation microscope is attached, and have a second arm unit, to which the laser treatment head is attached, the first and the second arm unit being adjustable relative to one another and preferably independently of one another.

Such a design enables the laser treatment head to be swivelled or otherwise moved into a non-use position, in which it does not interfere with the freedom of action of the physician working over the eye of the patient and looking through the operation microscope. Only if a laser treatment is to be performed on the eye, the physician can then move the laser treatment head under the operation microscope.

Insofar as the laser treatment head and the operation microscope are positionally adjustable relative to one another, it can be advantageous if the laser treatment head is lockable relative to the operation microscope or can otherwise be detachably coupled to the operation microscope once the laser treatment head has been moved under the operation microscope. This enables the laser treatment head to be fixed in position relative to the operation microscope, which fixing in position can therefore also be important, primarily, in order that the operating physician can maintain a reliable view of what is happening on and in the eye, through the observation passage provided in the laser treatment head.

For sterile working conditions, at least the operation microscope, or in any case a part thereof, can be covered by a sterile cover during the operation. The same applies to the laser treatment head, at least insofar as the latter is also to remain under the operation microscope, and therefore in the sterile working region, during a subsequent open intervention on the eye. If, on the other hand, the laser treatment head (without the operation microscope) can be moved out of the working region of the operating physician, it is possible to dispense with a sterile wrapping of the laser treatment head, in any case when all laser tasks are performed before the intervention on the open eye.

The stand arm arrangement can provide at least one rotational degree of freedom of movement or/and at least one translational degree of freedom of movement for the operation microscope and the laser treatment head, relative to the stand base in each case. In this case, it is possible to pass through at least a majority of the movement scope of the operation microscope and of the laser treatment head, relative to the stand base, by manual adjustment. If required, a drive arrangement, for example an electric motor-operated drive arrangement, which allows motor-operated adjustment, in particular for the purpose of fine positioning of the operation microscope and/or of the laser treatment head, can be provided additionally on the stand. However, the adjustment range provided by such a drive arrangement is preferably small relative to the available manual adjustment range.

According to one design, a method for performing an eye operation can comprise the following steps:
- providing an adjustable stand in an operation room, there being attached to the stand an operation microscope and a laser treatment head that emits pulsed, focussed laser radiation having radiation properties suited to the application of incisions in the human eye,
- positioning a patient on a treatment couch in a sterile region of the operation room,
- setting the stand into a first position, in which the laser treatment head is positioned in an observation beam path of the operation microscope, and an operating physician can observe, through the operation microscope and an observation passage of the laser treatment head, an eye of the patient to be operated upon,
- performing a laser treatment of the eye, by means of the laser radiation, in the first position of the stand,
- setting the stand into a second position, in which the laser treatment head is positioned outside the observation beam path of the operation microscope, and the operating physician can observe, solely through the operation microscope, the eye of the patient to be operated upon,
- performing further operation tasks on the eye, in the second position of the stand, without use of the laser radiation.

The invention is explained further in the following with reference to the appended drawings, wherein:
Figure 1 is a schematic representation of a first embodiment of an eye-surgery apparatus for laser-assisted intraocular eye operations,
Figure 2 is a schematic representation of a second embodiment of an eye-surgery apparatus for laser-assisted intraocular eye operations,
Figure 3 is a schematic representation of a third embodiment of an eye-surgery apparatus for laser-assisted intraocular eye operations.

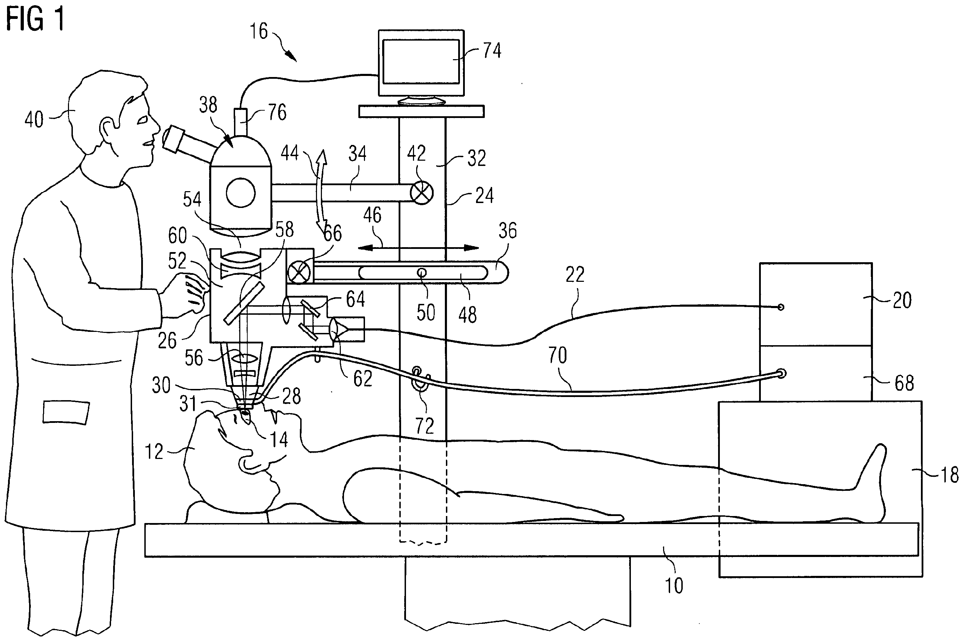

Reference is made first to Fig. 1. Set up at the operation station represented therein is a patient bed (patient couch) 10, on which, in the representation of Fig. 1, there lies a patient 12, having an eye 14 to be treated, which is represented merely schematically, and a laser system 16, which is suitable for producing incisions in the tissue of the patient's eye 14 by photodisruption. The laser system 16 comprises a laser source 20, which is disposed on a supporting frame 18 (for example, in the form of a shelf or table) and which contains e.g. a solid-state laser or a fibre laser and provides pulsed laser radiation. The laser radiation emitted by the laser source 20 is coupled into a flexible transmission fibre 22, via which the laser radiation is transmitted to a laser treatment head 26, which is held on a stand 24 and from which the laser radiation is applied to the patient's eye 14. The laser radiation emitted by the laser treatment head 26 has radiation properties suited to producing photodisruptions in the tissue of the patient's eye 14. For example, the pulse durations of the applied laser pulses are in the range of picoseconds or femtoseconds. In order to avoid excessively high pulse intensities on the transmission fibre 22, the pulse durations of the laser pulses coupled into transmission fibre 22 by the laser source 20 can be greater than the pulse durations of the laser pulses applied to the eye 14. For this purpose, a pulse stretcher (not represented in greater detail), which stretches the pulse durations of the laser pulses, for example to more than one picosecond, can be provided in the laser source 20. For the subsequent time compression of the laser pulses to the required, shorter pulse durations of, for example, femtoseconds or picoseconds, the transmission fibre itself can have corresponding compression properties, for which purpose, for example, a photonic hollow core fibre can be used (frequently designated as a PCF fibre, i.e. "photonic crystal fibre"). Alternatively, it is possible to use a transmission fibre without, or at least without significant, compression properties, for instance an LMA fibre, i.e. a transmission fibre having a large mode area (LMA = large mode area). A suitable compression element, for instance a transmission grating or a crystal including a chirped Bragg grating (not represented in greater detail), can then be provided in the laser treatment head 26 for the purpose of pulse compression.

Exchangeably attached to the laser treatment head 26 is a patient adapter (applicator) 28, which constitutes a mechanical interface to the patient's eye 14 and allows referencing of the eye 14 in relation to the laser treatment head 26. For this purpose, the adapter 28 has a contact element 30, which is transparent to laser radiation and through which the laser radiation is applied. On its side that faces towards the eye, the contact element 30 constitutes a contact surface against which the eye 14 is placed. In a manner known per se, the patient adapter 28 can be realized for coupling to a suction ring 31 to be placed beforehand on the eye 14.

In the exemplary case shown, the stand 24 is realized as a floor stand, which is preferably movable, and thus can be moved out of the operation room after an eye operation, to enable the operation room to be cleaned. Alternatively, the stand 24 can be a wall stand or ceiling stand, which is fixedly mounted on a wall or on the ceiling of the operation room. In each case, the stand 24 has a stand base 32, which, in the exemplary case shown in Fig. 1, is realized schematically as an upright column and which, in the case of a floor stand, is realized with rollers at its foot that can be locked if appropriate, or, in the case of a wall stand or ceiling stand, constitutes a support for mounting on the wall or ceiling. Attached to this stand base 32, generally, is a stand arm arrangement, which can be adjusted relative to the stand base in preferably a plurality of degrees of freedom (translationally and/or rotationally) and which, in the exemplary case shown, comprises two arm units 34, 36 that can be adjusted separately from one another. Attached to one of the arm units, in this case the arm unit 34, there is an operation microscope 38, which offers an operating physician 40, indicated schematically, an enlarged view of the operation region (the eye 14). The laser treatment head 26, on the other hand, is attached to the other arm unit (in this case, the arm unit 36). The arm units 34 - as in the simplified, schematic representation of Fig. 1 - can each be individual arms that can be adjusted pivotally or/and linearly in relation to the stand base 32. It is understood, however, that each of the arm units 34 can be a multi-arm structure composed of a plurality of arms, which are connected to one another in a jointed manner or/and through linear motion guides.

In Fig. 1, the arm unit 34 that carries the operation microscope 38 is shown to be pivotable about a horizontal pivot axis 42, relative to the stand base 32 (according to a double arrow 44), while the arm unit 36 that caries the laser treatment head 26 is adjustable in a horizontal direction, guided linearly in relation to the stand base 32 (as illustrated by a double arrow 46). In Fig. 1, the linear guidance of the arm unit 36 in relation to the stand base 32 is illustrated, in a purely schematic manner, by a peg and longitudinal slot arrangement, having a longitudinal slot 48 and a peg 50 guided therein. It need not be especially emphasized that this is a representation purely for the purpose of illustration, and that considerably more complex motion mechanisms can be provided for the purpose of motional guidance of the operation microscope 38 and of the laser treatment head 26 in a plurality of degrees of freedom of movement in relation to the stand base 32.

A characteristic of the embodiment of Fig. 1, however, is that the laser treatment head 26 can be moved, relative to the operation microscope 38, between a position of use and a non-use position. The position of use is represented in Fig. 1; in this position, the laser treatment head 26 is moved over the eye 14 to be treated, and can be docked onto the eye 14 through the use of the patient adapter 28. The laser treatment head 26 in this case is located between the eye 14 and the operation microscope 38. In order that the operating physician 40 can nevertheless observe, through the operation microscope 38, what is happening on the eye 14, the laser treatment head 26 constitutes an observation passage 52, which extends from an observation window 54 (formed by suitable observation optics, for example), facing towards the microscope 38, as far as the patient adapter 28, such that, when the laser treatment head 26 is in the position of use, the observation beam path of the operation microscope 38 extends through the observation passage of the laser treatment head 26 as far as the eye 14.

In the non-use position, on the other hand, which is not represented in greater detail in the drawing, the laser treatment head 26 is moved out of the observation beam path of the operation microscope 38, such that the operating physician 40, when looking through the operation microscope 38, has a direct view onto the eye 14. The laser treatment head 26 is then no longer located under the operation microscope 38 and, in particular, is at such a distance from the working region over the eye 14 that the operating physician 40 can perform the remaining operation tasks on the eye 14 in an unimpeded manner.

When the laser treatment head 26 is in the position of use, the observation beam path of the operation microscope 38 goes through various optical elements, which are provided in the laser treatment head 26 for the purpose of guiding or/and shaping the laser radiation. In particular, the observation beam path of the microscope 38 goes through a focussing optical system 56, for example in the form of an F-Theta objective lens, and, in the exemplary case shown, goes through a semi-transparent deflecting mirror 58. The optical elements for guiding and shaping the laser radiation are matched to the wavelength of the laser radiation used. For visible light, which reaches the operation microscope 38 through the observation passage 52, optical aberrations (for example, a chromatic dispersion) can therefore occur, to compensate which a compensating optical system 60 can be provided, in the laser treatment head 26.

Additionally accommodated in the laser treatment head 26 are a collimator lens 62 and a scanning arrangement, which is denoted in general by 64. The collimator lens 62 serves to collimate the divergent radiation bundle leaving the transmission fibre 22. The scanning arrangement 64 serves to shift the focus position of the focussed radiation bundle emerging from the laser treatment head 26, both in the direction of beam propagation (usually designated as the z direction) and in a plane transverse to the z direction (usually designated as the x-y plane). For the purpose of transverse scanning (i.e. in the x-y direction), the scanning arrangement can comprise, for example, in a manner known per se, a pair of galvanometrically controllable deflection mirrors, which can be tilted about axes that are perpendicular to one another. For the purpose of longitudinal scanning (i.e. in the z direction), on the other hand, the scanning arrangement can have, for example, a lens that is positionally adjustable or of variable refractive power, or an adaptive mirror. For miniaturization it is conceivable to provide, for example, as an alternative to a pair of galvanometric mirrors, an electrooptic crystal, by means of which a controlled x-y deflection of the focus position can likewise be achieved.

For the purpose of z displacement of the radiation focus it is also conceivable, alternatively, to realize the focussing optical system 56 so as to be adjustable in the direction of the radiation propagation (i.e. z direction).

Denoted at 66 in Fig. 1 is an additional pivot joint, which allows the laser treatment head 26 to be pivoted about a pivot axis that is perpendicular to the arrow direction 46 (i.e., in the representation of Fig. 1, about a pivot axis normal to the plane of the page).

For the purpose of controlling the laser source 20, the scanning arrangement 64 and, if appropriate, the focussing optical system 56, the laser system 16 comprises a control unit 68, which can be set up together with the laser source 20 on the supporting frame 18. For the purpose of transmitting electrical control signals from the control unit 68 to the laser treatment head 26, an electrical connecting cable, not represented in Fig. 1, runs between the two components. At the same time, the control unit 68 can include a pump arrangement, likewise not represented in greater detail, but known per se, having at least one vacuum pump. The vacuum generated by this pump arrangement can be transported, via a vacuum tube (or, if appropriate, a plurality of vacuum tubes) that can be connected to the control unit 68, to the suction ring 31, where the vacuum is used to suck the suction ring 31 onto the eye 14 and, if appropriate, also to suck the patient adapter 28 onto the suction ring 31. The vacuum tube 70 can run on the stand 24, through a guide 72, for instance through a guide clip 72, which is indicated schematically. The control intelligence contained in the control unit 68 also controls the pumping operation of the aforementioned pump arrangement.

The frame 18 is realized, advantageously, such that it can be taken out of the operation room with little effort. For this purpose, it can be realized as a rolling frame, or it can be fastened to the stand 24, such that it can be removed together with the stand 24 from the operation room. For example, the frame 18 can be attached to the stand base 32. If a wall stand or ceiling stand is used, the frame 18, alternatively, can be mounted on the wall or ceiling of the operation room, separately from the stand.

Further, in addition, a monitor 74 can be attached to the stand 24, for example to the stand base 32, on which monitor there can be visualized camera recordings that are recorded by means of a microscope camera 76 attached to the operation microscope 38. The physician 40 or his assisting personnel can thus follow the operation on the monitor 74.

The laser appliance 16 shown in Fig. 1 enables the physician 40, the patient 12 and the assisting personnel to remain in their positions in the sterile region of the operation room during the entire cataract operation (or other laser-assisted intraocular operation). The operation need not be interrupted after the laser treatment by means of the laser system 16. Instead, after use of the laser, the physician can continue working in an uninterrupted manner by means of an ultrasound device (not represented in greater detail in Fig. 1) and the other instruments required for extracting the crystalline lens and replacing it by an artificial lens and complete the surgery. After use of the laser, it is necessary only to move the laser treatment head 26 out of the working area of the physician 40, through use of the degrees of freedom of movement offered by the stand arm unit 36. The patient 12 need not be transferred to another bed, and the physician 40 need not leave the sterile region. Also, it is not necessary for the physician to change clothes. This saves a considerable amount of time.

It is understood that, even in the case of a design in which the laser treatment head 26 and the operation microscope 38 are positionally adjustable relative to one another (as represented in Fig. 1), the physician 40 can nevertheless decide, after use of the laser, to continue the operation without moving the laser treatment head 26 back out of its position of use into the non-use position. In other words, the laser treatment head 26 can remain under the operation microscope 38 even during the subsequent operation tasks. For this purpose, it can be appropriate to first remove the patient adapter 28, in order thus to create sufficient space under the laser treatment head 26 for the manual operation tasks in hand.

The position of use of the laser treatment head 26 can be, for example, a locking position, into which the arm unit 36 latches automatically when the laser treatment head 26 is moved into the position of use. If required, a motor-assisted fine positioning of the laser treatment head 26, for example in the vertical direction, can be possible in the position of use, in particular to facilitate docking of the patient adapter 28 to the suction ring 31 and to the eye 14. For this purpose, a suitable motor-operated drive means (not represented in greater detail), allowing a corresponding adjustment of the arm unit 36, can be provided on the stand 24.

On the other hand, the frame 18, with the control unit 68 and the laser source 20, can be disposed in a semi-sterile region of the operation room, at a sufficient distance from the sterile working region of the physician 40, and also remain there during the entire operation.

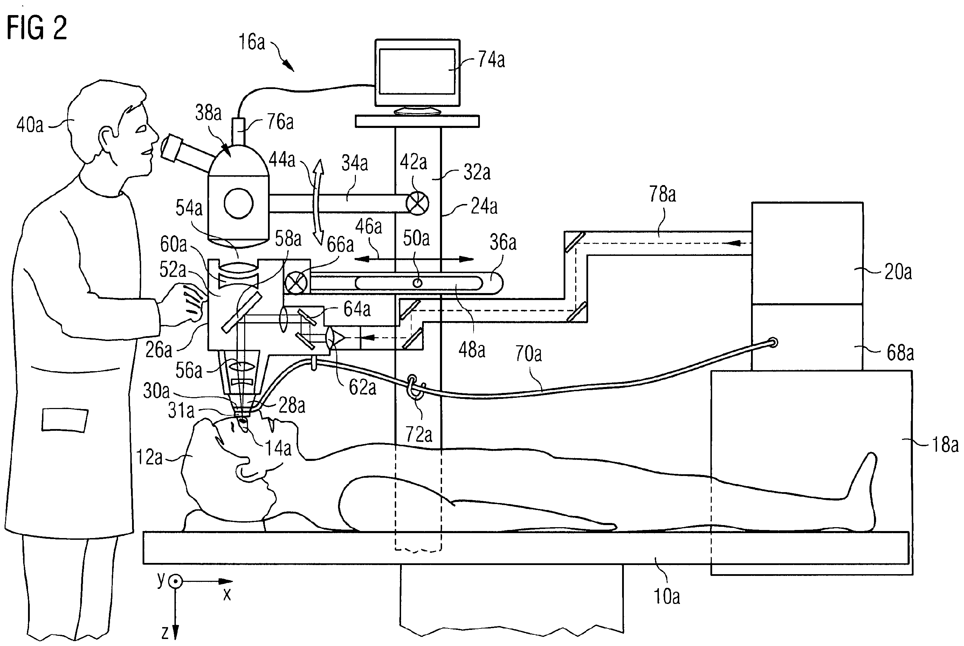

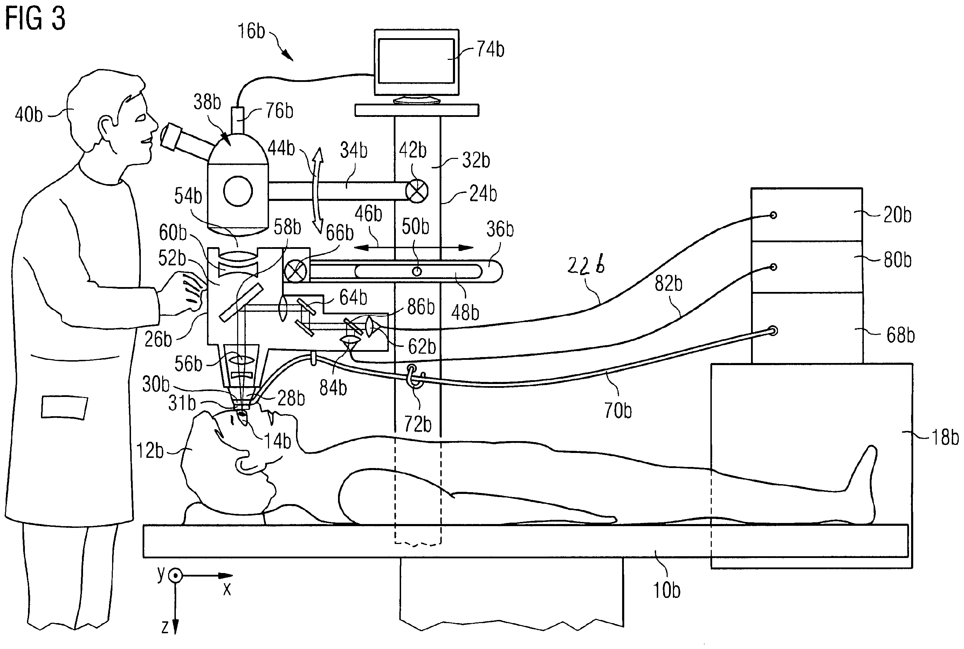

In the further Figures 2 and 3, components that are the same or have the same function are denoted by the same references as in Fig. 1, but suffixed with a lower-case letter. Unless otherwise stated in the following, for explanation of such components we refer to the preceding statements relating to Fig. 1.

The embodiment of Fig. 2 differs from that of Fig. 1, in essence, in the provision of a mirror jointed arm 78a for transporting the laser radiation from the laser source 20a to the laser treatment head 26a. The mirror jointed arm 78a offers a sufficient freedom of movement to allow the desired/required adjustability of the laser treatment head 26a relative to the stand 24a or/and of the arm unit 36a carrying the laser treatment head 26a, in relation to the stand base 32a, and not to impede such adjustability.

It may be desirable for the eye-surgery apparatus to be equipped with a diagnostic unit, in particular an imaging diagnostic unit, for example to enable the laser treatment of the patient's eye (for instance the capsulorhexis and the lens prefragmentation in the case of a laser-assisted cataract operation) to be performed in a precisely localized manner. For this purpose, the third embodiment shown in Figure 3 is equipped with an OCT measuring appliance, which comprises an OCT unit 80b disposed, together with the laser source 20b and the control unit 68b, on the frame 18b. OCT stands for optical coherence tomography. The OCT unit 80b can interferometrically overlay an emitted OCT measurement radiation with an OCT reflected radiation reflected from the patient's eye 14b and, from the thereby obtained interferometry data, generate a two-dimensional or three-dimensional image of the tissue structures of the eye 14b. The generated OCT image can be displayed, for example, on the monitor 74b. Alternatively, it is conceivable for the OCT unit 80b to be connected to a further monitor (not represented in greater detail), on which it can display the OCT image. If required, such a monitor can also be integrated into the OCT unit 80b.

In the embodiment shown in Figure 3, there is connected to the OCT unit 80b a further transmission fibre 82b, which is separate from the transmission fibre 22b and via which the OCT measurement radiation is transported from the OCT unit 80b to the laser treatment head 26b. In the laser treatment head 26b, the OCT measurement radiation goes through the scanning arrangement 64b and the focussing optical system 56b. It is coupled, via a collimator lens 84b and a semi-transparent mirror 86b, into the radiation propagation path that is provided, in the laser treatment head 26b, for the laser radiation transported via the transmission fibre 22b. The components of the OCT measurement radiation reflected at the eye 14b (i.e. the OCT reflected radiation) is routed on the same path to the transmission fibre 82b and, via the latter, to the OCT unit 80b.

In departure from the exemplary case shown in Figure 3, it is conceivable for one or both of the two transmission fibres 22b, 82b to be replaced by an appropriately movable mirror jointed arm (analogous to the embodiment of Figure 2). It is conceivable in this case, for example, to use a transmission fibre for one of the two radiation types (laser radiation, OCT measurement radiation) and, for the other radiation type, to use a mirror jointed arm for transporting the radiation to the laser treatment head 26b. Alternatively, it is conceivable to use two separate mirror jointed arms for transporting, respectively, one of the two radiation types.

In a further modification of Figure 3 it is conceivable to provide a common transport path to the laser treatment head 26b for both radiation types, either in the form of a common transmission fibre or in the form of a common mirror jointed arm. When provision is made for a common transport path for the laser radiation and the OCT measurement radiation (and also the OCT reflected radiation) it may be provided that the laser radiation and the OCT measurement radiation are not emitted simultaneously. If simultaneous operation of the laser source 20b and of the OCT unit 80b is required, it may be beneficial to use separate transport media for the laser radiation and the OCT measurement radiation. The wavelength of the laser radiation and the wavelength of the OCT measurement radiation may be relatively close to one another, for example - to give a number example that is not limiting in any way - 1030 nm for the laser radiation and 1060 nm for the OCT measurement radiation. Alternatively, the wavelengths of the laser radiation and the OCT measurement radiation may be comparatively far apart from one another, for example 1030 nm for the laser radiation and 800 nm for the OCT measurement radiation.

In respect of the generation of the OCT measurement radiation, use can be made of a measurement radiation source that is separate from the laser source 20b and that, expediently, is integrated into the OCT unit 80b. It is also conceivable, however, to generate the OCT measurement radiation by means of the laser source 20b, such that, in this case, a single radiation source suffices for generation of both types of radiation.

## Claims

1. Apparatus for eye surgery, comprising
- a stand (24) having a stand base (32) that is movable or realized for mounting on a wall or ceiling, and having a stand arm arrangement (34, 36) that is manually adjustable, at least partially, relative to the stand base,
- an operation microscope (38) attached to the stand arm arrangement, the operation microscope being pivotable about a horizontal pivot axis (42),
- a laser appliance, which provides pulsed, focussed laser radiation having radiation properties suited to the application of incisions in the human eye (14), the laser appliance having a laser source (20), a control unit (68) for controlling the laser source and a laser treatment head (26) attached to the stand arm arrangement (34, 36) and emitting the laser radiation, the laser treatment head being pivotable about an additional pivot joint (66) that is parallel to the horizontal pivot axis (42), a flexible transmission fibre (22) or a jointed beam transport arm for transporting the laser radiation to the laser treatment head, and an electrical connecting cable for transmitting electrical control signals from the control unit to the treatment head,
the laser treatment head being positioned or positionable in an observation beam path of the operation microscope and providing a passage (52) for an observation beam going along the observation beam path.

2. Apparatus according to Claim 1, wherein the operation microscope (38) and the laser treatment head (26) are coupled or coupleable to one another relative to the stand base (32) for the purpose of common positional adjustment.

3. Apparatus according to either one of the preceding claims, wherein the operation microscope (38) and the laser treatment head (26) are positionally adjustable relative to one another, such that the laser treatment head is moveable into and out of the observation beam path of the operation microscope.

4. Apparatus according to Claim 3, wherein the stand arm arrangement has a first arm unit (34), to which the operation microscope (38) is attached, and has a second arm unit (36), to which the laser treatment head (26) is attached, the first and the second arm unit being adjustable relative to one another.

5. Apparatus according to any one of the preceding claims, wherein the stand arm arrangement (34, 36) provides at least one rotational degree of freedom of movement or/and at least one translational degree of freedom of movement for the operation microscope (38) and the laser treatment head (26), relative to the stand base (32) in each case.

6. Apparatus according to any one of the preceding claims, comprising an optical-coherence, interferometric measuring appliance (80b) having a source for measurement radiation, a flexible transmission fibre (82b) or a jointed beam transport arm, which transmission fibre or which beam transport arm is connected to the laser treatment head (26b) and transports the measurement radiation to the laser treatment head, the laser treatment head providing, for the measurement radiation, a radiation propagation path in which one or more optical scanner components (64b) and a focussing optical system (56b) are disposed.

7. Apparatus according to Claim 6, wherein the measuring appliance (80b) operates according to a method of optical coherence tomography.

8. Apparatus according to Claim 6 or 7, wherein the apparatus comprises a common transmission fibre or a common beam transport arm for transporting the laser radiation and the measurement radiation.

9. Apparatus according to Claim 6 or 7, wherein the apparatus comprises separate beam transport units (22b, 82b) for transporting the laser radiation and the measurement radiation.

## Patentansprüche

1. Vorrichtung für die Augenchirurgie, umfassend
- einen Ständer (24) mit einer Ständerbasis (32), die beweglich ist oder zur Befestigung an einer Wand oder Decke realisiert ist, und mit einer Ständerarmanordnung (34, 36), die bezüglich der Ständerbasis zumindest teilweise manuell verstellbar ist,
- ein Operationsmikroskop (38), das an der Ständerarmanordnung befestigt ist, wobei das Operationsmikroskop um eine horizontale Schwenkachse (42) schwenkbar ist,
- ein Lasergerät, das eine gepulste fokussierte Laserstrahlung mit Strahlungseigenschaften, die für das Anbringen von Inzisionen im menschlichen Auge (14) geeignet sind, bereitstellt, wobei das Lasergerät eine Laserquelle (20), eine Steuereinheit (68) zur Steuerung der Laserquelle und einen Laserbehandlungskopf (26), der an der Ständerarmanordnung (34, 36) befestigt ist und die Laserstrahlung aussendet, wobei der Laserbehandlungskopf um ein zusätzliches Drehgelenk (66), das parallel zur horizontalen Schwenkachse (42) ist, schwenkbar ist, eine flexible Übertragungsfaser (22) oder einen gelenkig verbundenen Strahlentransportarm zum Transportieren der Laserstrahlung zum Laserbehandlungskopf, und ein elektrisches Verbindungskabel zur Übertragung von elektrischen Steuersignalen von der Steuereinheit zum Behandlungskopf, aufweist,
wobei der Laserkopf in einem Beobachtungsstrahlengang des Operationsmikroskops positioniert oder positionierbar ist und einen Durchgang (52) für einen Beobachtungsstrahl, der entlang des Beobachtungsstrahlengangs verläuft, bereitstellt.

2. Vorrichtung nach Anspruch 1, wobei das Operationsmikroskop (38) und der Laserbehandlungskopf (26) bezüglich der Ständerbasis (32) zur gemeinsamen Positionseinstellung miteinander verbunden oder verbindbar sind.

3. Vorrichtung nach einem der vorhergehenden Ansprüche, wobei das Operationsmikroskop (38) und der Laserbehandlungskopf (26) in ihrer Position in Bezug aufeinander verstellbar sind, so dass der Laserbehandlungskopf in den und aus dem Beobachtungsstrahlengang des Operationsmikroskops bewegbar ist.

4. Vorrichtung nach Anspruch 3, wobei die Ständerarmanordnung eine erste Armeinheit (34), an der das Operationsmikroskop (38) befestigt ist, und eine zweite Armeinheit (36), an der der Laserbehandlungskopf (26) befestigt ist, aufweist, wobei die erste und die zweite Armeinheit in Bezug aufeinander verstellbar sind.

5. Vorrichtung nach einem der vorhergehenden Ansprüche, wobei die Ständerarmanordnung (34, 36) mindestens einen Drehbewegungsfreiheitsgrad und/oder mindestens einen Verschiebungsbewegungsfreiheitsgrad für das Operationsmikroskop (38) und den Laserbehandlungskopf (26) jeweils in Bezug auf die Ständerbasis (32) bereitstellt.

6. Vorrichtung nach einem der vorhergehenden Ansprüche, umfassend eine interferometrische optische Kohärenz-Messvorrichtung (80b) mit einer Quelle zur Messung von Strahlung, einer flexiblen Übertragungsfaser (82b) oder einem gelenkig verbundenen Strahlentransportarm, wobei die Übertragungsfaser oder der Strahlentransportarm mit dem Laserbehandlungskopf (26b) verbunden ist und die Messstrahlung zum Laserbehandlungskopf transportiert, wobei der Laserbehandlungskopf für die Messstrahlung einen Strahlungsfortpflanzungsweg bereitstellt, in dem ein oder mehrere optische Scannerkomponenten (64b) und ein fokussierendes optisches System (56b) angeordnet sind.

7. Vorrichtung nach Anspruch 6, wobei die Messvorrichtung (80b) nach einem Verfahren der optischen Kohärenz-Tomographie arbeitet.

8. Vorrichtung nach Anspruch 6 oder 7, wobei die Vorrichtung eine gemeinsame Übertragungsfaser oder einen gemeinsamen Strahlentransportarm für den Transport der Laserstrahlung und der Messstrahlung umfasst.

9. Vorrichtung nach Anspruch 6 oder 7, wobei die Vorrichtung getrennte Strahlentransporteinheiten (22b, 82b) für den Transport der Laserstrahlung und der Messstrahlung umfasst.

## Revendications

1. Appareil pour chirurgie de l'oeil, comprenant
- un support (24) ayant une base (32) de support qui est mobile ou réalisée pour être montée sur un mur ou à un plafond, et possédant un agencement de bras (34, 36) de support qui est réglable manuellement, au moins partiellement, par rapport à la base de support,
- un microscope d'opération (38) fixé à l'agencement de bras de support, le microscope d'opération pouvant pivoter sur un axe de pivot horizontal (42),
- un appareil électrique laser, qui fournit un rayonnement laser focalisé puisé ayant des propriétés de rayonnement adaptées à l'application d'incisions dans l'oeil humain (14), l'appareil électrique laser ayant une source laser (20), une unité de commande (68) pour commander la source laser et une tête de traitement laser (26) fixée à l'agencement de bras (34, 36) de support et émettant le rayonnement laser, la tête de traitement laser pouvant pivoter sur un joint (66) de pivot supplémentaire qui est parallèle à l'axe (42) de pivot horizontal, une fibre de transmission flexible (22) ou un bras de transport de faisceau à joint pour transporter le rayonnement laser vers la tête de traitement laser, et un câble de connexion électrique pour transmettre des signaux de commande électrique de l'unité de commande à la tête de traitement,
la tête de traitement laser étant positionnée ou positionnable dans un trajet de faisceau d'observation du microscope d'opération et fournissant un passage (52) pour un faisceau d'observation longeant le trajet de faisceau d'observation.

2. Appareil selon la revendication 1, dans lequel le microscope d'opération (38) et la tête de traitement laser (26) sont accouplés ou peuvent être accouplés l'un à l'autre par rapport à la base (32) de support à des fins d'ajustement commun de position.

3. Appareil selon l'une quelconque des revendications précédentes, dans lequel le microscope d'opération (38) et la tête de traitement laser (26) sont ajustables en position l'un par rapport à l'autre, de sorte que la tête de traitement laser soit mobile vers et hors du trajet de faisceau d'observation du microscope d'opération.

4. Appareil selon la revendication 3, dans lequel l'agencement de bras de support a une première unité de bras (34), à laquelle est fixé le microscope d'opération (38), et a une seconde unité de bras (36), à laquelle est fixée la tête de traitement laser (26), la première et la seconde unité étant ajustables l'une par rapport à l'autre.

5. Appareil selon l'une quelconque des revendications précédentes, dans lequel l'agencement de bras (34, 36) de support permet au moins un degré de liberté de mouvement en rotation ou/et au moins un degré de liberté de mouvement en translation pour le microscope d'opération (38) et la tête de traitement laser (26), par rapport à la base (32) de support dans chaque cas.

6. Appareil selon l'une quelconque des revendications précédentes, comprenant un appareil de mesure interférométrique à cohérence optique (80b) ayant une source pour un rayonnement de mesure, une fibre de transmission flexible (82b) ou un bras de transport de faisceau à joint, laquelle fibre de transmission ou lequel bras de transport de faisceau étant connecté à la tête de traitement laser (26b) et transportant le rayonnement de mesure vers la tête de traitement laser, la tête de traitement laser fournissant, au rayonnement de mesure, un trajet de propagation de rayonnement dans lequel sont disposés un ou plusieurs éléments (64b) de scanner optique et un système optique de focalisation (56b).

7. Appareil selon la revendication 6, dans lequel l'appareil électrique de mesure (80b) fonctionne selon un procédé de tomographie à cohérence optique.

8. Appareil selon la revendication 6 ou 7, l'appareil comprenant une fibre de transmission commune ou un bras de transport de faisceau commun pour transporter le rayonnement laser et le rayonnement de mesure.

9. Appareil selon la revendication 6 ou 7, l'appareil comprenant des unités de transport de faisceau distinctes (22b, 82b) pour transporter le rayonnement laser et le rayonnement de mesure.
